(19)

Europäisches Patentamt

European Patent Office

Office européen des brevets

(11) **EP 2 570 131 A1**

(12) **EUROPEAN PATENT APPLICATION**
published in accordance with Art. 153(4) EPC

(43) Date of publication:
**20.03.2013 Bulletin 2013/12**

(21) Application number: **11779999.9**

(22) Date of filing: **11.05.2011**

(51) Int Cl.:
*A61K 36/07* (2006.01)     *A61P 33/02* (2006.01)

(86) International application number:
**PCT/BR2011/000147**

(87) International publication number:
**WO 2011/140623 (17.11.2011 Gazette 2011/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.05.2011 BR PI1001570**
**11.05.2010 BR PI1002067**

(71) Applicants:
• **Universidade Federal De Minas Gerais - UFMG**
**31270-901 Belo Horizonte MG (BR)**
• **Minasfungi De Brasil**
**31872-403 Belo Horizonte, MG (BR)**

(72) Inventors:
• **FERRAZ COELHO, Eduardo Antonio**
**31110-150**
**Belo Horizonte, MG (BR)**
• **CÉSAR BENTO RÉGIS, Wiliam**
**30640-350**
**Belo Horizonte, MG (BR)**

• **GARCIA VALADARES, Diogo**
**30380-725**
**Belo Horizonte, MG (BR)**
• **PEREIRA TAVARES, Carlos Alberto**
**31340-500**
**Belo Horizonte, MG (BR)**
• **SALLES MOURA FERNANDES, Ana Paula**
**30430-000**
**Belo Horizonte, MG (BR)**
• **SILVANO DE OLIVEIRA, Jamil**
**Belo Horizonte, MG (BR)**
• **MATOS SANTORO, Marcelo**
**30360-310**
**Belo Horizonte, MG (BR)**

(74) Representative: **Winter, Brandl, Fürniss, Hübner, Röss, Kaiser, Polte - Partnerschaft**
**Alois-Steinecker-Strasse 22**
**85354 Freising (DE)**

(54) **FORMULATION AGAINST LEISHMANIASIS AND USE THEREOF**

(57) The present invention refers to pharmaceutical formulations obtained from the aqueous extract of the fungus *Agaricus blazei* and its purified fractions for the treatment of leishmaniasis. More particularly, the present invention discloses formulations preferably for topic and oral use, in form of solid, semi-solid and liquid pharmaceutical formulations selected from a group consisting of gel, cream, ointment, pastes, emulsions in general, solutions, tablets and capsules for the treatment of canine and human cutaneous and visceral leishmaniasis.

Figure 1

**Description**

The present invention refers to pharmaceutical formulations obtained from the aqueous extract of the fungus *Agaricus blazei* and its purified fractions for the treatment of leishmaniasis. More particularly, the present invention discloses formulations preferably for topic and oral use, in form of solid, semi-solid and liquid pharmaceutical formulations selected from a group consisting of gel, cream, ointment, pastes, emulsions in general, solutions, tablets and capsules for the treatment of canine and human cutaneous and visceral leishmaniasis.

The mushroom *Agaricus blazei (A. blazei)* is an aerobic fungus that has the potential to degrade organic matter rich in cellulose, hemicellulose and lignin to obtain energy. Said mushroom was reclassified by Wasser (2002) as *Agaricus brasilienses,* however, the name *Agaricus blazei* have been more used in the scientific literature due to biotechnological and medicinal aspects of the mushroom, as in most of the marketed products thereof [WASSER, S.P. Medicinal mushrooms as a source of antitumor and immunomodulating polysaccharides. Appl. Microbiol. Biotechnol., v. 60, p. 258-74, 2002); AMAZONAS, M.A.L.A. *Agaricus brasiliensis (= Agaricus blazei* ss. Heinem.): last view on the controversial issue of the taxonomic identity of one of the most promising mushrooms in the world market. In: International Symposium on mushrooms in Brazil, Brasilia, DF. p. 78-80, 2004].

Its nutritional and medicinal value, combined with unique characteristics such as its flavor, the almond fragrance and the excellent texture, make it particularly suitable for many culinary applications, being one of the most valued cultivated mushrooms in the world market (STIJVE, T., AMAZONAS, M.A.L., GILLER, V. Flavor and taste components of Agaricus blazei ss. Heinem: a new gourmet and medicinal mushroom. Deutsche Lebensmittel-Rundschau, Stuttgart, v. 98, p. 448-453, 2002).

Among the various benefits of the *A. blazei* to the human body are the control of type II diabetes, arterial hypertension and osteoporosis, calcium uptake through ergosterol, cancer and AIDS treatment. Some of these properties are related to substances in the food compound of *A. blazei,* such as o beta-D-glucan, cerebrosides, steroids, ergosterol and fatty acids (MIZUNO, M., MORIMOTO, M. MINATO, K., TSUCHIDA, H. Polysaccharides from Agaricus blazei stimulate lymphocyte T-cell subsets in mice. Biosci. Biotechnol. Biochem., v. 62, p. 434-437, 1998). Its most prominent and studied biological activity is the immunostimulation, which protects against infection and helps in eliminating malignant cells (URBEN, A.F. Morphological and physiological characterization in accesses of Agaricus blazei and A. sylvaticus. In: V Latin American Mycology Congress, Brasilia, p. 203-205, 2005).

The main mechanism of action of *A. blazei* appears to be related to the biological activity of polysaccharides, especially the so-called $(1 \rightarrow 6)$ - $(1 \rightarrow 3)$-β-D-glucan, found in the fruiting body. In countries like Japan, Russia, China and United States there were extracted different polysaccharides with antitumor activities from the fruiting body and mycelia of several species of medicinal mushrooms. Most of these polysaccharides do not directly act on tumor cells, but have indirect antitumor effects due to activation of different immune response pathways of the host. The biological action of the consumption of mushrooms is mainly due to the increase of stimulation and activation of macrophages (WASSER, S.P. & WEIS, A.L. Medicinal properties of substances occurring in higher basidiomycetes mushrooms: Current perspectives (review). Int. J. Med., v. 1, p. 31-62, 1999).

The immunostimulatory activity of *A. blazei* has been characterized by various research groups (MIZUNO, T. & HAGIWARA, T. Antitumor activity and some properties of water-soluble polysaccharides from "Himematsutake", the fruiting body of Agaricus blazei Murrill. Agricult. Biologic. Chemist., Tokyo, v. 54, p. 2889-2896, 1990a; MIZUNO, T., INAGAKI, R., KANAO, R. Antitumor activity and some properties of water-insoluble hetero-glycans from "Himematsutake", the fruiting body of Agaricus blazei Murill. Agric. Biol. Chem., 54, 2897-2905, 1990b; EBINA, T. & FUJIMIYA, Y. Antitumor effect of a peptide-glucan preparation extracted from Agaricus blazei in a double-grafted tumor system in mice. Biotherapy, v. 11, p. 259-65, 1998; KUO, Y.C., HUANG, Y.L., CHEN, C.C. Cell cycle progression and cytokine gene expression of human peripheral blood mononuclear cells modulated by Agaricus blazei. J. Lab. Clin. Med., v. 140, p. 176-87, 2002). Studies using soluble fractions in water at 100 °C have indicated its inhibitory action on the growth of solid tumors and tumor cells, as well as reduction of metastases. The fractions studied have specifically led to a significant increase in the proliferation of T and B lymphocytes *in vitro* and increased NK cell activity (DONG Q, YAO J, YANG XT, FANG JN. Structural characterization of a water-soluble beta-D-glucan from fruiting bodies of Agaricus blazei Murr. Carbohydr Res., v. 3, p.1417-21, 2002; FUJIMIYA Y; SUZUKI Y; OSHIMAN K.; Selective tumoricidal effect of soluble proteoglucan extracted from the basidiomycete, Agaricus blazei Murill, mediated via natural killer cell activation and apoptosis. Cancer Immunol Immunother., v. 46, p. 147-59, 1998). *In vivo* Studies have demonstrated the immunostimulatory activity of said fractions on T lymphocytes, induction of increased expression of cytokines and interleukins (such as IL-6 and IL-1β), differentiation of B cells, increased antibody production and of the expression of CR3 (Mac-1), CD25 and B7-1 receptors, considered cell surface markers, which recognize tumor cells and pathogens (NAKAJIMA, A., ISHIDA, T., KOGA M. Effect of hot water extract from Agaricus blazei Murill on antibody-producing cells in mice. Int. Immunopharmacol., v. 2, p. 1205-11, 2002). Other studies have demonstrated the inhibition of tumor cells by *A. blazei* through immune activation depending on the alternative complement pathway, formation of an opsonizing complex with C3bi in human serum (SHIMIZU, S., KITADA, H., YOKOTA, H. Activation of the alternative complement pathway by Agaricus blazei murill. Phytomedic., v. 9, p. 536-45, 2002).

Sorimachi et al. (2001) have observed that *A. blazei* components are able to activate macrophages, resulting in an increased production of cytokines such as TNF-α, IL-8 nitric oxide (NO). An aqueous extract of *A. blazei,* in turn, has increased the mRNA expression of IL-1β and IL-6, both in peritoneal macrophages and in the spleen cells from mice stimulated *in vitro* (SORIMACHI, K., AKIMOTO, K., IKEHARA, Y. Secretion of TNF-α, II-8 and nitric oxide by macrophages activated with Agaricus blazei murrill fractions in vitro. Cell Struct. Func., v. 26, p. 103-108, 2001; NAKAJIMA, A., ISHIDA, T., KOGA M. Effect of hot water extract from Agaricus blazei Murill on antibody-producing cells in mice. Int Immunopharmacol., v. 2, p. 1205-11, 2002). Different fractions extracted from the aqueous extract of *A. blazei,* originated from mycelia and fruiting body culture, have induced secretion of cytokines such as IL-8 and TNF-α, by the macrophages from bone marrow of mice and increased *in vitro secretion of NO* (Sorimachi et al., 2001). The stimulation of NK cells, the generation of selective cytotoxic cells and the induction of apoptosis in tumor cells *in vitro* by proteoglycan extract extracted from *A. blazei* have also been demonstrated (FUJIMIYA Y; SUZUKI Y; OSHIMAN K.; Selective tumoricidal effect of soluble proteoglucan extracted from the basidiomycete, Agaricus blazei Murill, mediated via natural killer cell activation and apoptosis. Cancer Immunol Immunother., v. 46, p. 147-59, 1998). In addition to its antitumor activity, studies have demonstrated that mushroom polysaccharides have antimicrobial, antiviral, hepatoprotective, antifibrotic, hypoglycemic and hypocholesterolemic properties (SAKAGAMI, H., AOKI, T., SIMPSON, A. Induction of immunopotentiation activity by a protein-bound polysaccharide, PSK (Review). Anticancer Res., v. 11, p. 993-1000, 1991; SORIMACHI, K., NIWA, A., YAMAZAKI, S. Antiviral activity of water-solubilized lignin derivatives in vitro. Agric. Biol. Chem., v. 54, p. 1337-1339, 1990; OOI, V.E.C. Hepatoprotective effect of some mushrooms. Phytother. Res. West Sussex, v. 10, p. 536-538, 1996; PARK, E.J., KO, G., KIM, J. Antifibrotic effects of a polysaccharide extracted from Ganoderma lucidum, glyclyrrhizin, and pentoxifyline in rats with cirrhosis induced in biliary obstruction. Biol. Pharm. Bull., v. 20, p. 417-420, 1997; HIKINO, H. & MIZUNO, T. Hypoglycemic actions of some heteroglycans of Ganoderma lucidum fruit bodies. plant Med., v. 55, p. 385, 1989; CHEUNG, P.C.K. The hypocholesterolemic effect of extracellular polysaccharide from the submerged fermentation of mushroom. Nutr. Res., v. 16, p. 1953-1957, 1996).

The present invention describes the use of *A. blazei* aqueous extract and its protein and non-protein fractions, purified therefrom, for the topical and oral treatment of canine and human cutaneous and visceral leishmaniasis.

Leishmaniasis is a disease caused by protozoan parasites of the genus *Leishmania,* which can cause from simple skin lesions, with spontaneous healing, to the visceral form, fatal when untreated (DESJEUX, P. Leishmaniasis: current situation and new perspectives. Comp. Immunol. Microbiol. Infect. Dis., v. 27, p. 305-318, 2004).

The *Leishmania* parasite has two main morphological forms: promastigote and amastigote. The promastigote forms are elongated, flagellated, mobile, with a single core and kinetoplast, located between the anterior portion and the core, which multiply in the digestive tract of the vector-insect. The amastigote forms are rounded forms, with rudimentary flagellum, rod-shaped kinetoplast, and they multiply within cells of the phagocytic-monocytic system in the mammalian host (Grimaldi, G.Jr. & Tesh, R.B. Leishmaniasis of the New World: current concepts and implications for future research. Clin. Microbiol. Res., v. 6, p. 230-250, 1993).

Different species of mammals, among them rodents and canines are natural reservoirs of the parasite and serve as a source of infection for the vector. The dog can be identified as the main domestic host for visceral leishmaniasis (VL). Foxes and wolves are sylvatic reservoirs of the disease, while marsupials and rodents can be reservoirs of species that cause cutaneous leishmaniasis (CL). The female sandflies are the vectors of the disease (Diptera: Psicodidae), belonging to the genus *Lutzomyia* in the Americas and *Phlebotomus* in the Old World countries (SACKS, D. & KAMHAWI, S. Molecular aspects of parasite-vector and vector-host interactions in leishmaniasis. Annu. Rev. Microbiol., v. 55, p. 453-483, 2001).

The vector infection occurs when the female feeds from an infected host, when, along with blood, macrophages containing amastigotes are ingested. Then there is the release of amastigotes in the digestive tract of the vector, which rapidly undergo morphological and biochemical changes and evolve to the procyclic promastigote form and, then, to the metacyclic promastigote. The mammalian host is infected when bitten by an infected vector; when it injects the metacyclic promastigotes of *Leishmania* under the skin of the host. These forms are opsonized by proteins of the complement system or antibodies and/or phagocytized by macrophages, forming phagolysosomes, where they transform into amastigotes. After successive replication by binary fission, the parasite can cause the lysis of macrophages and the consequent release thereof. The amastigotes can be phagocytosed by new macrophages, ending the cycle of infection in the mammalian host, or can be ingested by another vector, thus, completing the biological cycle of the parasite (Grimaldi, G.Jr. & Tesh, R.B. Leishmaniasis of the New World: current concepts and implications for future research. Clin. Microbiol. Rev., v. 6, p. 230-250, 1993; Sacks, D. & Sher, A. Evasion of innate immunity by parasitic protozoa. Nat. Immunol., v. 3, p. 1041-1047, 2002.).

Several Leishmania species have been described and can be considered causes of different clinical forms of the disease. The CL is characterized by a diversity of clinical manifestations and disease causing species. In Brazil, it can occur due to infection by *Leishmania braziliensis, L. guyanensis, L. amazonensis, L. shawi, L. laisoni* and *L. naiffi* and, clinically, it can be localized or disseminated. The cutaneous form of leishmaniasis is characterized by the existence of a single lesion with raised edges, of granular base and painless. Vegetating, verrucous or infiltrative lesions are less frequent (MARZOCHI, M.C., MARZOCHI, K.B., CARVALHO, R.W. Visceral leishmaniasis in Rio de Janeiro. Parasitol. Today, v. 10, p. 34-37, 1994; DESJEUX, P. Leishmaniasis: current situation and new perspectives. Comp. Immunol. Microbiol. Infect. Dis., v. 27, p. 305-318, 2004; Silveira FT, Lainson R, Corbett CE. Clinical and immunopathological spectrum of American cutaneous leishmaniasis with special reference to the disease in Amazonian Brazil: a review. Mem. Inst. Oswaldo Cruz. V. 99, p. 239-251, 2004). There are still cases of mucocutaneous leishmaniasis, which occur in several countries and are caused by species *L. braziliensis, L. panamensis, L. guyanensis* and *L. amazonensis.* In this form, the lesions have infiltrative character which can ulcerate and bleed. The cutaneous-disseminated form occurs due to infection by species *L. aethiopica,* in Africa or by species *L. amazonensis* and *L. mexicana* in South American countries. It is a form in which the lesions can appear as plaques, nodules, sometimes vegetating, but rarely ulcerate. The lesions disseminate in exposed regions of the body and said situation can be associated with inefficiency or absence of an effective immune response by the host (WEIGLE, K. & SARAVIA, N.G. Natural history, clinical evolution, and the host-parasite interaction in New World cutaneous leishmaniasis. Clin. Dermatol., v. 14, p. 433-450, 1996.; Desjeux, 2004).

Data from the World Health Organization indicate that there is incidence of said disease in about 88 countries, of which 72 are developing countries. The estimated annual incidence is about 1.0 to 1.5 million new cases of CL and about 500,000 cases of VL. Approximately 350 million people are at infection risk areas and it is estimated an increase in the number of cases throughout the world in the coming years (SHAW, J. The leishmaniasis-survival and expansion in a changing world. A mini-review. Mem. Inst. Oswaldo Cruz. 541-7, 2007).

Another aspect that has shown clinical and epidemiological importance is the co-infection between HIV virus and *Leishmania.* Leishmaniasis can modify the progression of the disease caused by HIV and facilitate immunosuppression caused by virus which leads to progression of the disease in several countries worldwide.

The treatment of leishmaniasis in human patients should be conducted to avoid mortality caused by VL and reduce morbidity caused by the disfiguring lesions observed in the more severe forms of CL. Usually, treatment involves the application of local or systemic antimonial pentavalent compounds, including sodium stibogluconate (Pentostam®, Glaxo Wellcome, England) and N-methyl meglumine antimoniate (Glucantime®, Rhône Poulenc Rorer, France) are the most used (CARVALHO, P.B.; ARRIBAS, M.A.G.; FERREIRA, E.I. Leishmaniasis. What do we know about its chemotherapy? Braz. J. Pharmac. Sci., v. 36, p. 69-96, 2000. FRANKE, E.D.; WIGNALL, F.S.; CRUZ, M.E.; ROSALEZ, E.; TOVAR, A.A.; LUCAS, C.M.; LIANOS-CUENTAS, A.; BERMAN, J.D. Efficacy and toxicity of sodium stibogluconate for mucosal leishmaniasis. Ann. Intern. Med., v. 113, p. 934-940, 1990. HERWALDT, B.L. Leishmaniasis. Lancet, v. 354, p. 1191-1199, 1999).

In Brazil, Glucantime® has been used as drug of choice. However, such drug can interact with sulfhydryl cellular protein of the host causing loss of function and/or forming complexes with ribonucleosides, which makes the action of the product unspecific in relation to infected cells and those uninfected. Second-line drugs, such as amphotericin B, have been recommended in cases of intolerance or resistance to conventional treatment and should be administered n a hospital environment (SUNDAR, S., SINGH, A., AGARWAL, D., RAI, M., AGARWAL, N., CHAKRAVARTY, J. Safety and efficacy of high-dose infusions of a preformed amphotericin B fat emulsion for treatment of Indian visceral leishmaniasis. Am. J. Trop. Med. Hyg., v. 80, p. 700-3, 2009).

Human treatment with pentavalent antimonials has several limitations that reduce patient adherence to it, among them the long duration of treatment (20 to 40 days, with daily applications of product), routes of drug administration (intramuscular or intravenous) and the severe side effects caused by the administration of the drugs. High daily doses, required in the course of treatment, can cause fatigue, arthralgias, myalgias and also renal (chronic renal failure), liver (cirrhosis) and heart (arrhythmia) toxicity. It can be also cited the difficulty of transporting the patients, who usually live in rural areas, to the most specialized health centers, in addition to the high costs of the drugs (CARVALHO, P.B.; ARRIBAS, M.A.G.; FERREIRA, E.I. Leishmaniasis. What do we know about its chemotherapy? Braz. J. Pharmac. Sci., v. 36, p. 69-96, 2000. GROGL, M.; MARTIN, R.K.; ODUOLA, A.M.J.; MILHOUS, W.K.; KYLE, D.E. Characteristics of multidrug resistance in Plasmodium and Leishmania: detection of P-glycoprotein-like components. Am. J. Trop. Med. Hyg., v. 45, p. 98-111, 1991. TAVARES CA, FERNANDES AP, MELO MN. Molecular diagnosis of leishmaniasis. Expert Rev Mol Diagn. v. 3, p. 657-667, 2003). These aspects hinder patients' adherence to treatment, so that it is common to abandon or discontinue it, which leads to increased parasite resistance to the used drugs.

Cunninghan (2002) reported that about 10 to 25% of patients with VL treated with pentavalent antimonials were resistant to the treatment or presented recurrences. Sundar (2001) also reported that a significant percentage of patients with visceral disease caused by L. donovani were resistant to treatment with Pentostam®(CUNNINGHAM, A.C. Parasitic adaptive mechanisms in infection by Leishmania. Exp. Mol. Pathol., v. 72, p. 131-141, 2002. For these cases, pentamidine and amphotericin B can be used, despite the high toxicity and high cost of these drugs (Grimaldi & Tesh, 1993).

Dogs are important reservoirs in the domestic cycle of VL and are considered the main source of infection for sandflies due to the strong prevalence of canine infection when compared to human infection. Infected dogs, even if asymptomatic, have plenty of parasites in the skin which favors infection of the insect vector from this reservoir and consequently the transmission to humans. It should be noted that human infection has no impact on the biological cycle as an important source of infection (Tesh, 1995 and WHO, 2003). This fact, associated with the lethality of VL in the absence of treatment, led the Ministry of Health of Brazil to adopt the elimination of dogs when seropositive for Leishmania antigens, as infection control measure. However, serological methods such as IFA and ELISA, commonly used to diagnose the disease in the dog, can have different sensitivity and specificity and, thus, the real infection rates can be underestimated. This allows the maintenance of infected animals, which is one of the reasons for the failure to control the disease (Tesh, 1995).

Drugs available in the market for the treatment of canine VL such as alopurinol, the pentavalent antimonials and amphotericin B are not viable as a measure to control the disease because they have high price and often treated and clinically cured dogs suffer recurrences, thus remaining sources of infection for the vector (Tesh, 1995). Moreover, the use of these drugs in the mass treatment of canine VL brings another concern, which is the possible increased risk of selecting strains which are resistant to those drugs that are already used to treat humans (Reithinger *et al.,* 2002).

According to the various facts mentioned above, it is noted the need of conducting new researches focused on the discovery of new alternative therapies for treating humans and dogs against the disease.

Thus, the identification of new compounds/products/drugs that are less toxic to patients, more economically viable, a fact which is not observed in the currently used; and whose administration route is improved in order to cause the least discomfort possible to the patients becomes very attractive. In this context, the use of the fungus *Agaricus blazei,* in the form of its aqueous extract and/or its purified fractions present in pharmaceutical formulations containing said products, it is proposed the minimization and/or solution of said problems.

When working with the aqueous extract, the goal is to incorporate it into a formulation, and for such, the first step is to put it in a formulation compatible with its constituents which maintains its stability and assist its pharmacological action. Furthermore, there are studies in the literature relating the non-toxicity of the fungus when used in human patients by oral route, so that no side or toxic effect was evidenced in individuals. Thus the administration of the fungus extract and its fractions topically (for CL) cannot be considered invasive and will certainly be much less uncomfortable for the patient when compared to the routes normally used for antimonials, in this case, intramuscular and intravenously. Additionally, pharmaceutical formulations in solid and/or liquid form will also be developed for oral administration in the case of VL.

Besides all these advantages, there is still the fact that the fungus is abundant in our flora and currently being released by ANVISA for commercialization in form of a nutraceutical.

## Brief description of the Figures

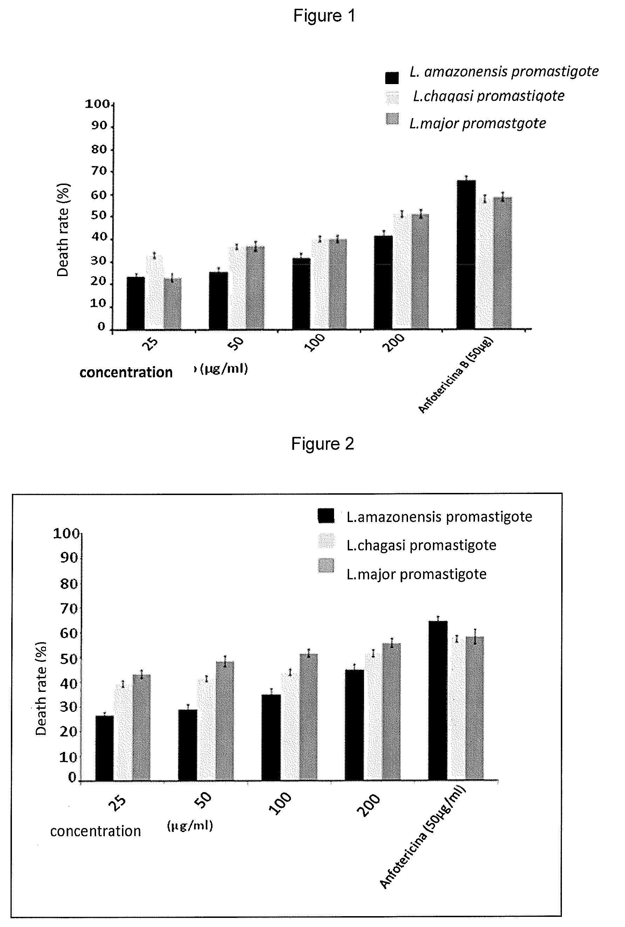

**Figure 1:** Leishmanicidal activity of aqueous extract of *Agaricus blazei* on stationary phase promastigotes of three *Leishmania* species. Parasites ($4 \times 10^5$) were incubated with varying concentrations (25 a 200 $\mu$g/mL) of the aqueous extract for 24 hours at 25°C. The viability of the parasites was determined by assays using the MTT reagent. The bars represent the average and standard deviation of 3 species.

**Figure 2:** Leishmanicidal activity of aqueous extract of *Agaricus blazei* on stationary phase amastigote-like forms of three Leishmania species Parasites ($4 \times 10^5$) were incubated with varying concentrations (25 a 200 $\mu$g/mL) of the aqueous extract for 24 hours at 25°C. Thereafter, the viability of the parasites was determined by assays using the MTT reagent. The bars represent the average and standard deviation of 3 species.

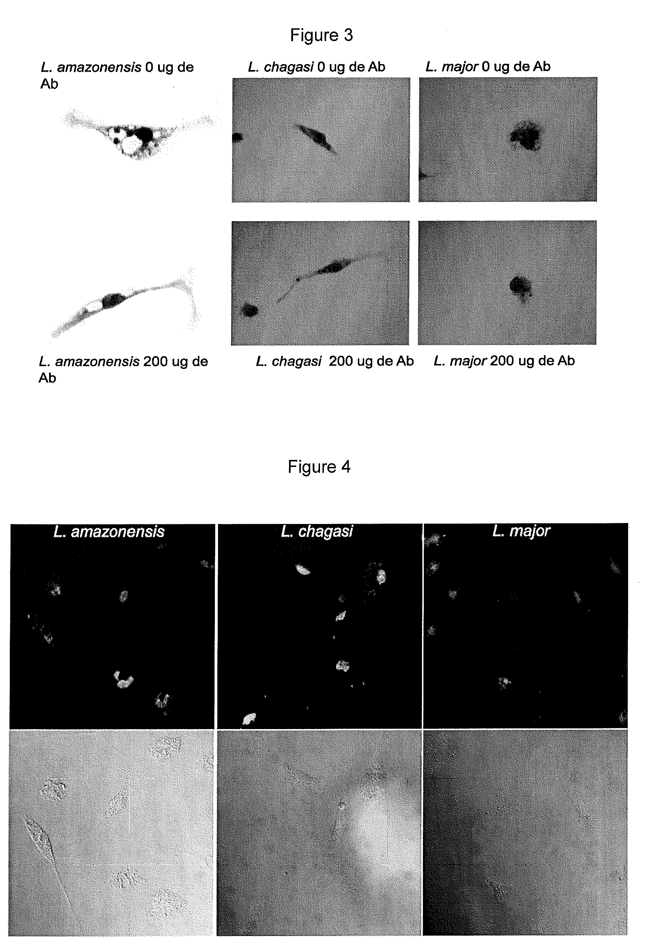

**Figure 3:** Inhibition of infection of peritoneal macrophages by stationary phase promastigotes of *L. amazonensis, L. chagasi* and *L. major.* The parasites ($4 \times 10^6$) were treated with 200 $\mu$g/mL of the aqueous extract of the fungus and incubated for 1h at 25°C and then put to infect macrophages (ratio of 10 *Leishmanias* for 1 macrophage) (B). As control, untreated parasites were infected (A). The data is representative of three experiments with similar results.

**Figure 4:** Inhibition of infection of peritoneal macrophages by stationary phase amastigote-like forms of *L. amazonensis, L. chagasi* and *L. major,* after treatment of parasites with the aqueous extract of fungus *Agaricus blazei.* The parasites were treated with 200 $\mu$g/mL of the fungus extract and incubated with peritoneal macrophages adhered to sterile coverslips in the proportion of 10 *Leishmanias* for each macrophage. As control, untreated parasites were used in the infection of macrophage cells. The results are representative of three experiments with similar results.

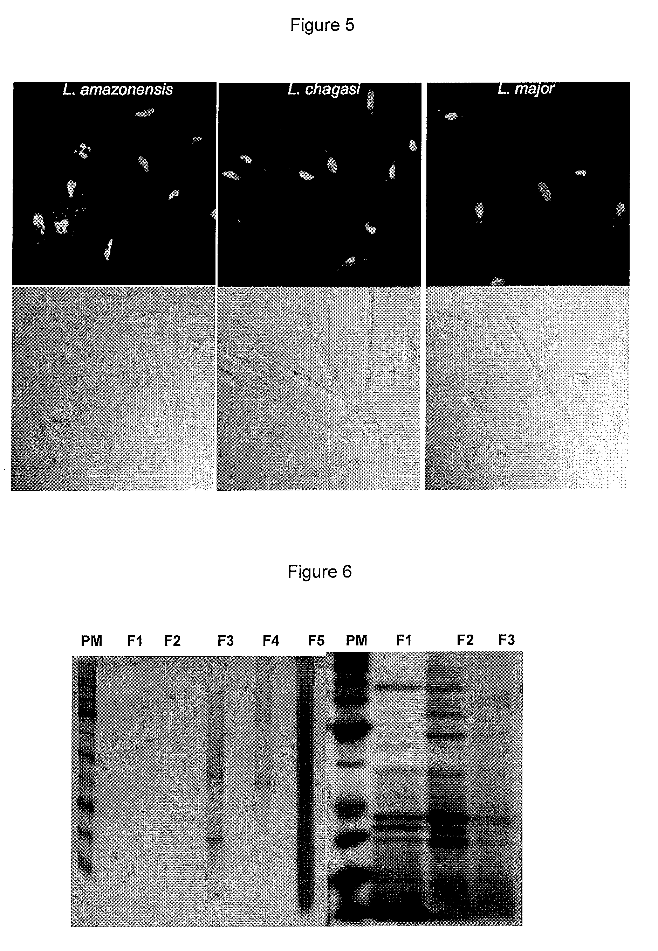

**Figure 5:** Treatment of infected macrophages. Peritoneal macrophages were infected with stationary growth phase promastigotes of *L. amazonensis, L. major* and *L. chagasi,* in the proportion of 10 parasites for each macrophage and treated for 48 hours at 25 °C with 200 $\mu$g/mL of the aqueous extract of the fungus *Agaricus blazei* (A). As control of the experiment, cells were infected and received no treatment (B).

**Figure 6:** Polyacrylamide gel electrophoresis in 10% of the fractions of the aqueous extract of the fungus *Agaricus blazei.* The fractions are indicated as: **F1**: < 3.0 kDa; **F2**: between 3.0 and 10.0 kDa; **F3**: between 10 and 50 kDa; **F4**: between 50 and 100 kDa and **F5**: above 100 kDa. The samples were diluted in sample buffer under non-reducing, homogenized conditions and applied on the gel. The electrophoretic run was performed for 4 h at 80 Volts. The gel was stained with silver and photographed. **MW** corresponds to the standard molecular weight (Pharmacia Biotech®).

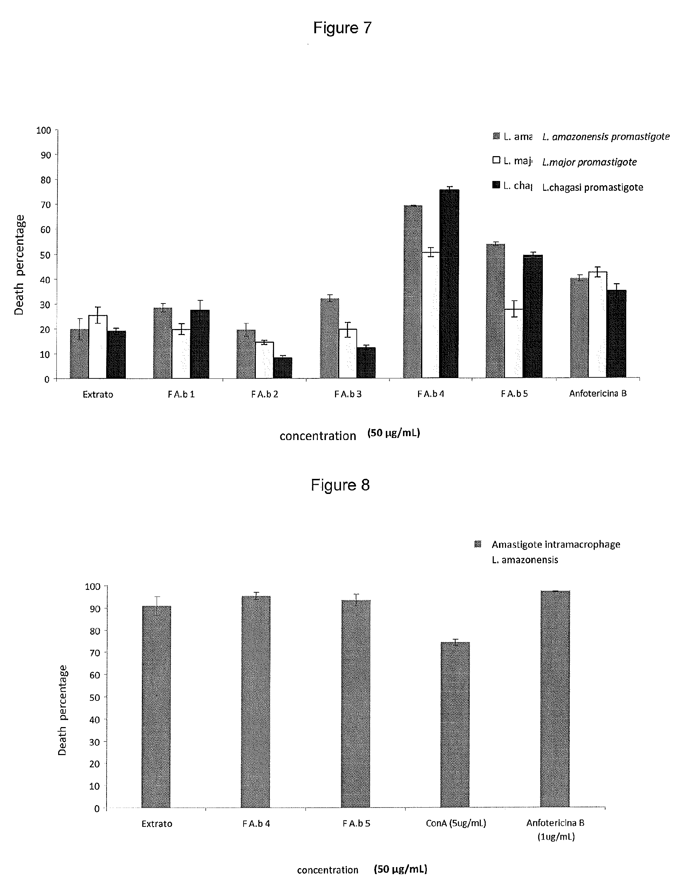

**Figure 7:** Leishmanicidal activity of the purified fractions on stationary growth phase promastigotes of *L. amazonensis, L. chagasi* and *L. major.* The experiment was conducted by the cell viability protocol by using the MTT reagent and the concentration of 50 μg/mL of the aqueous extract of the fungus and the purified fractions were used. As control it was used amphotericin B at a concentration of 50 μg/mL. The bars indicate the average and standard deviation of the experimental groups. The graph is representative of three experiments with similar results.

**Figure 8:** Leishmanicidal activity of purified fractions F4 and F5 of the fungus *A, blazei.* Peritoneal macrophages were infected at the proportion of 10 parasites for 1 cell, for 4 h and at 25°C. Thereafter, the cultures were washed and maintained for 24 h. Then, the infected macrophage cultures were treated for 48 h with the fractions F4 or F5. The experiment was performed in duplicate and there were quantified 100 macrophages per slide, whereby a percentage of the death of parasites within macrophages is determined.

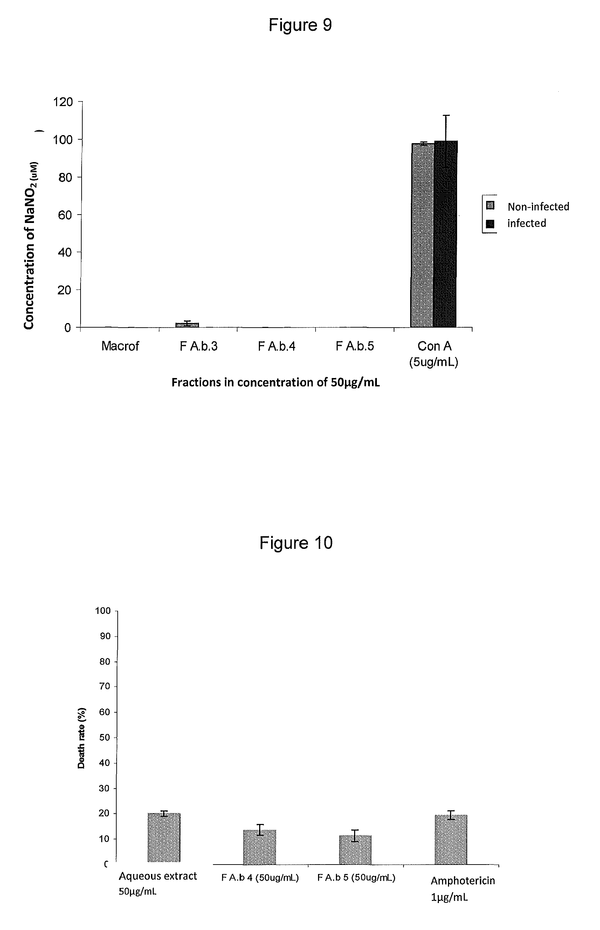

**Figure 9:** Dosage of nitric oxide (NO) in macrophages treated with fractions F3, F4 and F5. Murine peritoneal macrophages ($5x10^5$) were treated with 50 μg/mL of fractions F3, F4 or F5 for 48 hours at 25°C. Subsequently, the NO production was determined by the Griess reaction. As control it was used concanavalin A (ConA). The bars represent the average and standard deviation of three experiments with similar results.

**Figure 10:** Cytotoxicity of fractions F4 and F5 purified from the aqueous extract of fungus *A. blazei* on murine peritoneal macrophages. Macrophages were treated for 24 h and at 25°C with fractions F4 and F5. Cell viability was assessed by MTT assay.

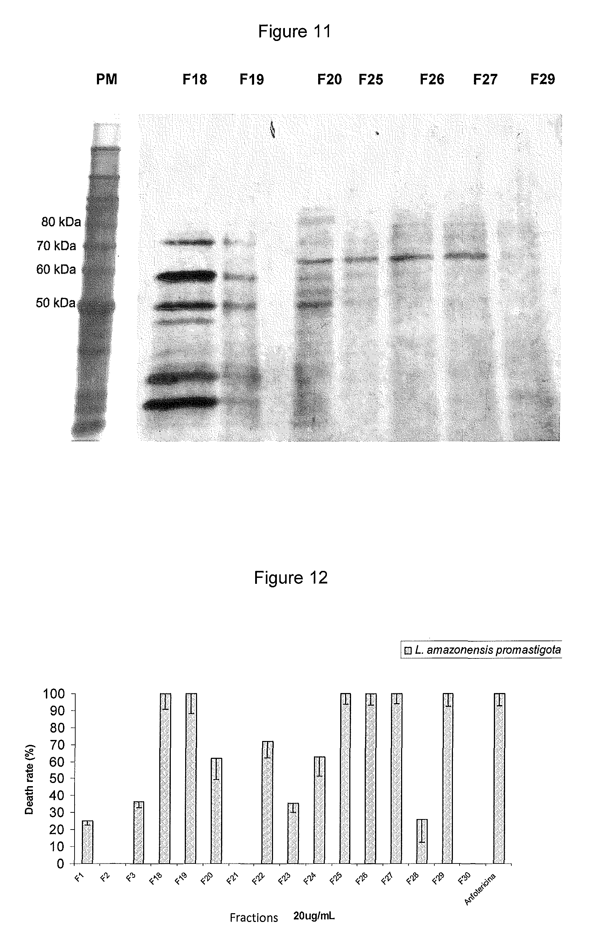

**Figure 11:** Polyacrylamide gel electrophoresis in the concentration of 10% of the fractions purified from fraction F5 by ion exchange chromatography. The samples were diluted in sample buffer under non-reducing, homogenized conditions and applied on the gel. The electrophoretic run was performed at 80 Volts for 4h. The gel was stained with silver and photographed. **MW corresponds to the standard molecular weight (Pharmacia Biotech®).**

**Figure 12:** Leishmanicidal activity of fractions purified from fraction F5 on stationary phase promastigotes of *L. amazonensis.* The experiment was conducted by the cellular viability protocol by using the MTT reagent and the concentration of 20 μg/mL of the fractions was used. As control it was used amphotericin B at a concentration of 50 μg/mL. The bars indicate the average and standard deviation of the experimental groups. The graph is representative of three experiments with similar results.

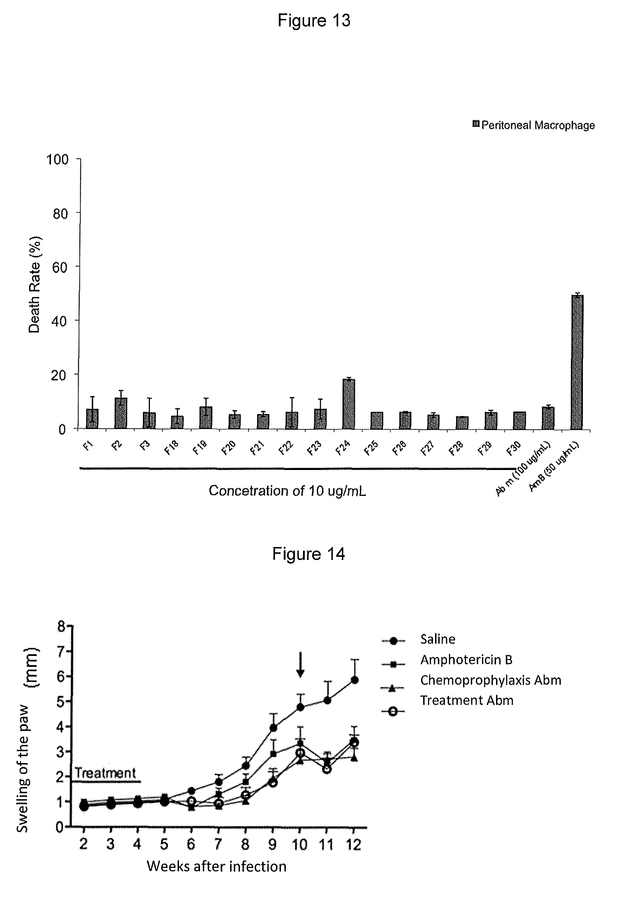

**Figure 13:** Cytotoxicity of fractions purified by ion exchange from fraction F5 on murine peritoneal macrophages. Macrophages were treated for 24 h and at 25°C with the new purified fractions (10 μg/mL). Cell viability was assessed by MTT assay.

**Figure 14:** Average size of foot lesions of infected BALB/c mice challenged with *L. amazonensis* and treated with aqueous extract of *Agaricus blazei.*

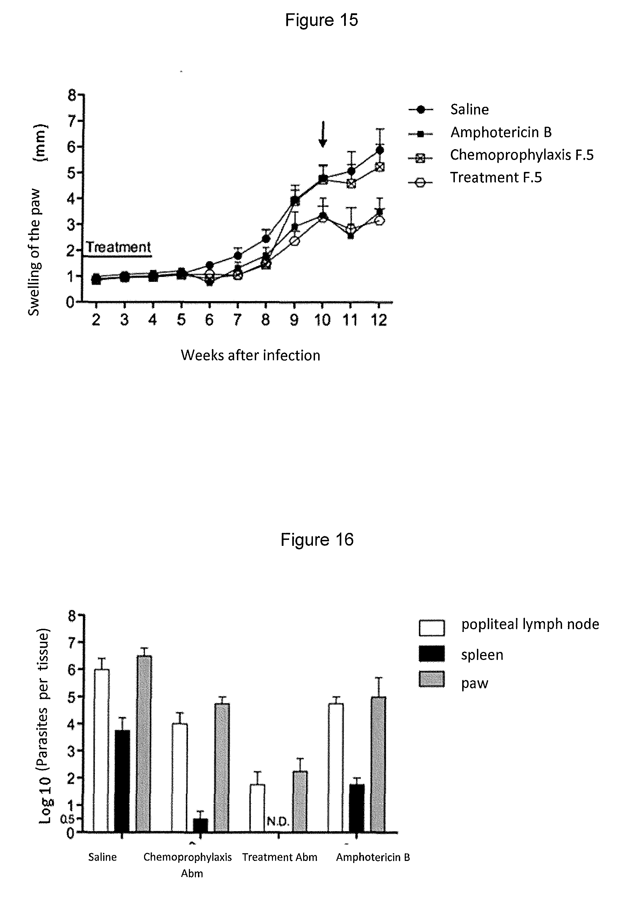

**Figure 15:** Average size of foot lesions of infected BALB/c mice challenged with *L. amazonensis* and treated with the fraction F5 purified with the aqueous extract of fungus *Agaricus blazei.*

**Figure 16:** Parasite load in BALB/c mice infected with *L. amazonensis* and subjected to different treatments with the aqueous extract of *Agaricus blazei.*

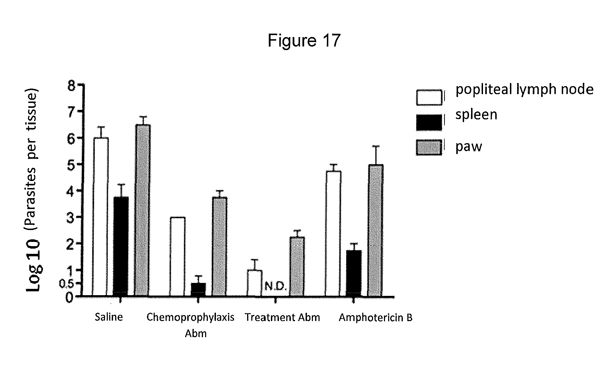

**Figure 17:** Parasite load in BALB/c mice infected with *L. amazonensis* and subjected to different treatments with fraction F5 of the aqueous extract of *Agaricus blazei.*

## Detailed description of the technology

The present invention is characterized by the use of aqueous extract of *Agaricus blazei* and its derived protein and non-protein fractions, combined with pharmaceutically acceptable excipients in the treatment of canine and human Cutaneous and Visceral Leishmaniasis. More particularly, the present invention discloses compositions preferably for topic and oral use, in form of solid, semi-solid and liquid pharmaceutical formulations selected from a group consisting of gel, cream, ointment, pastes, emulsions in general and solutions, tablets and capsules.

Said compositions can be administered by oral, intramuscular, intravenous, intraperitoneal, subcutaneous, transdermal route or as devices than can be implanted or injected; but they are preferably administered topically.

The technology proposed herein can be better understood through the following, non-limitating examples:

## Example 1 - Culture of Parasites

There were used the strains IFLA/BR/1967/PH-8 of *L. amazonensis,* MHOM/BR/1970/BH46 of *L. chagasi* and MHOM/IL/1980/Friedlin of *L. major.* The parasites were cultivated in complete Schneider's culture medium, which consists of the Schneider's medium (Sigma) supplemented with 20% inactivated fetal bovine serum (Sigma), 20 mM de L-glutamine, 50 μg/mL gentamycin, 200 U/mL penicilin and 100 μg/mL streptomycin at pH 7.4. The parasites were kept in culture at 25°C, so that the cultures were replated to maintain the strains and to obtain stationary growth phase promastigote forms.

**Example 2 - Preparation of aqueous extract of *Agaricus blazei***

For the preparation of aqueous extracts of *Agaricus blazei,* it is weighed about 1 gram of the fungus, and triturated in 1 mL of Tris-HCl 10 mM buffer, pH 7.0. After 1 h of incubation at 4°C, the extract was centrifuged at 9000 rpm (SORVAL LC5C centrifuge) for 1 h 30 minutes and the supernatant was recovered and its concentration estimated by the Bradford method (Bradford, 1976).

**Example 2.1- - Biological tests carried out with aqueous extract of the fungus**

**Leishmanicidal activity assay with aqueous extract of *Agaricus blazei***

The leishmanicidal activity of aqueous extract of the fungus *Agaricus blazei* on stationary phase amastigote-*like* forms of three different Leishmania species was tested.

In a cell culture plate of 96 wells (Nunc, Nunclon®), $4 \times 10^5$ promastigote and amastigote-*like* forms of species *Leishmania amazonensis, L. major* and *L. chagasi* were incubated with 25 to 200 μg/mL of the aqueous extract of the fungus in RPMI-PR medium for a final volume of 100 μL, for 24 h at 25°C. Then it was added 50 uL of MTT reagent *(Thiazoly Blue Tetrazolium Bromide* 98%; in stock concentration of 5 mg/mL) and the plate was incubated for 4 hours at 25°C. The cells were analyzed on a microscope to verify the formation of formazan crystals. Shortly after it was added 60 μL of a SDS 10%/HCL 0.1 M solution to solubilize formazan crystals and the plate was incubated for 18 h. The absorbance readings were then performed using a spectrophotometer at a wavelength of 570 nanometers (nm) (see Figures 1 and 2; Table 1 and 2).

Through the absorbance readings collected in experiments, Tables 1 and 2 were generated to represent the percentage of leishmanicidal activity of aqueous extract against the three *Leishmania* species tested in two manners. The calculation of the percentage of death of the parasites was based in the formula below, using the absorbance readings:

$$\text{Death rate} = \frac{(\text{D.O. }_{570nm}\text{ controle} - \text{D.O. }_{570nm}\text{ sample})}{\text{D.O. }_{570nm}\text{ control}} \times 100$$

**Table 1:** Average percentage of death of parasites using the aqueous extract of the fungus *Agaricus blazei* in different concentrations on the stationary phase promastigotes of *L. amazonensis, L. chagasi* and *L. major.* The data is representative of three experiments with similar results.

| Concentration.(μg/mL) | % Death *L. amazonensis* | % Death *L. major* | % Death *L. chagasi* |
|---|---|---|---|
| 25 | 23,7 | 23,24 | 33,24 |
| 50 | 25,67 | 37,2 | 37 |
| 100 | 31,86 | 40,41 | 40,41 |
| 200 | 41,76 | 51,46 | 51,46 |

**Table 2:** Average percentage of death of the parasites using the aqueous extract of fungus *Agaricus blazei* in different concentrations on the amastigote-*like* forms of *L. amazonensis, L. chagasi* and *L. major.* The data is representative of three experiments with similar results.

| Concentration (μg/mL) | % Death *L. amazonensis* | % Death *L. major* | % Death *L. chagasi* |
|---|---|---|---|
| 25 | 26,44 | 43,2 | 39,28 |
| 50 | 28,95 | 48,4 | 41 |
| 100 | 35 | 51,6 | 44 |
| 200 | 45 | 55,6 | 51,57 |

The effective concentrations to disable 50% of the *Leishmanias* (CE$_{50}$) were determined based on the results of leishmanicidal activity on promastigote and amastigote-like forms of *Leishmania* and are shown in Table 3.

**Table 3:** Effective concentration (EC50) of the aqueous extract of *Agaricus blazei* on promastigote and amastigote-*like* forms of *Leishmania* species

| Parasites | Effective concentration (EC$_{50}$) |
|---|---|
| Promastigotes of *L. amazonensis* | 136 |
| *Amastigote-like forms of L. amazonensis* | 124 |
| Promastigotes of *L. chagasi* | 105 |
| *Amastigote-like forms of L. chagasi* | 99 |
| Promastigotes of *L. major* | 138 |
| *Amastigote-like of L. major* | 89 |

**Effectiveness of the extract of the fungus in inhibiting the entry of parasites in mammalian macrophages**

To verify the effectiveness of the extract of the fungus in the entry of parasites in mammalian macrophages, promastigote and amastigote-*like* forms of *L. amazonensis, L. chagasi* and *L. major* were treated with the aqueous extract of the fungus and subsequently incubated with peritoneal macrophages derived from BALB/c mice at a proportion of 10:1. The parasites (4x10$^6$) were treated with 200 $\mu$g/mL of the aqueous extract of the fungus and incubated for 1h at 25°C and, then, put to infect macrophages (proportion of 10 *Leishmanias* for 1 macrophage) (B). As control, untreated parasites were infected (A). The data is representative of three experiments with similar results.

The quantification of the infected macrophages was made and the number of parasites per infected macrophage determined with the use of a composed microscope. The results are shown in figures 3 (promastigotes) and 4 (amastigote-*like*).

Thereafter the percentage of the number of macrophages infected by promastigote and amastigote-*like* forms and the ratio between the number of parasites per infected macrophage were determined. The data are shown in Tables 4 and 5.

**Table 4:** Percentage of number of macrophages infected with stationary phase promastigotes of *L. amazonensis, L. chagasi* and *L. major* and the ratio between the number of parasites per infected macrophages after treatment with the aqueous extract of the fungus *Agaricus blazei*.

| | % infected $\Phi$ | N° of parasites/ $\Phi$ |
|---|---|---|
| *L. amazonensis* | 84 | 12 |
| *L. amazonensis +200 $\mu$g/mL Ab* | 42 | 5 |
| *L. chagasi* | 77 | 9.2 |
| *L. chagasi + 200 $\mu$g Ab* | 62 | 4 |
| *L. major* | 64 | 5,4 |
| *L. major + 200 $\mu$g Ab* | 31 | 2,8 |

**Table 5:** Percentage of number of macrophages infected with amastigote-*like* forms of *L. amazonensis, L. chagasi* and *L. major* and the ratio between the number of parasites per infected macrophages after treatment with the aqueous extract of the fungus *Agaricus blazei*.

| | % infected $\Phi$ | N° of parasites/ $\Phi$ |
|---|---|---|
| *L. amazonensis* | 60 | 3,7 |
| *L. amazonensis +200 $\mu$g/mL Ab* | 35,8 | 1.9 |
| *L. chagasi* | 62 | 6 |
| *L. chagasi + 200 $\mu$g Ab* | 37 | 3,3 |

(continued)

| | % infected Φ | N° of parasites/ Φ |
|---|---|---|
| *L. major* | 65,6 | 6 |
| *L. major + 200 μg Ab* | 31 | 3,6 |

**Effect of the extract in infected macrophages**

Peritoneal macrophages were infected with *Leishmania* and subsequently treated with the aqueous extract of the fungus to verify the reduction of the infection in parasitized cells.

Peritoneal macrophages obtained from BALB/c mice were infected with stationary growth phase promastigotes of *L. amazonensis, L. major* and *L. chagasi* in the proportion of 10 parasites for each macrophage and treated for 48 hours at 25°C with 200 μg/mL of the aqueous extract of the fungus *Agaricus blazei* (A). As control, cells were infected and received no treatment (B). The results are shown in figure 5.

**Example 2.1 - Obtaining protein fractions from the aqueous extract of *Agaricus blazei***

For the preparation and extraction of protein fractions from the aqueous extract of the fungus *Agaricus blazei,* approximately 28 grams of the mushroom (the fruiting body is used), fresh and clean, were added in 50 mL of milli-Q water supplemented with 50 μL of protease inhibitor cocktail (SIGMA, CODE P8340). The material was homogenized in an ice bath with the aid of a mortar and pestle. Subsequently, the content has remained at rest at 4°C for 1 h and was filtered on filter paper to remove non-solubilized material.

The material was centrifuged at 10.000 rpm for 10 minutes at 4°C, and the supernatant was collected and centrifuged again in an Amicon column of 100.000 Daltons (Da) at a speed of 6.000 rpm for 45 minutes at 4°C. Thereafter, the material retained on the filter was removed and the remainder thereof was transferred to a new Amicon of 50.000 Da and centrifuged at 6.000 rpm for 30 minutes at 4°C. The retained material was collected and the remainder was passed to a new Amicon tube of 10.000 Da. The samples were centrifuged at 6.000 rpm for 30 minutes at 4°C and the material retained was removed, the remainder being applied in an Amicon of 3.000 Da. The same centrifugation procedure was repeated and the material retained in the membrane for 3.000 Da was removed, the remainder of the material being lyophilized.

Subsequently, the samples were quantified by the Lowry method and used in the coming biological assays (Table 6).

**Table 6 -** Obtaining protein fractions from the aqueous extract of the fungus *Agaricus blazei* per Amicon column. The protein fractions were separated by centrifugation gradient and quantified by the Lowry method.

| | molecular weight (Daltons) | Final concentration (mg/mL) |
|---|---|---|
| **F1** | Less than 3.000 | 0.8 |
| **F2** | Between 3.000 and 10.000. | 2.3 |
| **F3** | Between 10.000 and 50.000. | 6.57 |
| **F4** | Between 50.000 and 100.000. | 2.5 |
| **F5** | Above 100.000 | 44.0 |

**Polyacrylamide gel electrophoresis in 10% of the purified fractions of the aqueous extract of the fungus *Agaricus blazei.***

After purification in Amicon columns, the new fractions were subjected to SDS-PAGE gel electrophoresis at 10% to verify their protein profile, as shown in figure 6, in which 2 gels are shown.

**Example 2.2-: Leishmanicidal activity of the purified new fractions on stationary growth phase promastigotes of *L. amazonensis, L. chagasi* and *L. major.***

The fractions were tested as to their leishmanicidal activity on stationary growth phase promastigotes of *L. amazonensis, L. chagasi* and *L. major* and the results are shown in Figure 7.

The experiment was conducted by the cellular viability protocol by impregnating the MTT reagent and the concentration of 50 μg/mL of the aqueous extract of the fungus and the purified fractions were used. As control it was used amphotericin B at a concentration of 50 μg/mL (Figure 7).

Through the absorbance readings collected in the previous experiment, a table was generated to represent the percentage of leishmanicidal activity of aqueous extract of the fungus and its purified protein fractions on stationary growth phase promastigote forms of the three *Leishmania* species. The data are shown in Table 7.

**Table 7:** Average percentage of death of stationary phase promastigote forms of *L. amazonensis, L. chagasi and L. major* using aqueous extract of the fungus *Agaricus blazei* and its purified fractions. Amphotericin B was used as control of the experiment. The results indicate the standard average $\pm$ deviation of experimental groups. The data shown are representative of three experiments with similar results.

|  | *L. amazonensis* | *L. major* | *L. chagasi* |
|---|---|---|---|
| **Aqueous extract** | 20,08 $\pm$ 4,27 | 25,46 $\pm$ 3,20 | 18,98 $\pm$ 1,27 |
| **F1** | 28,41 $\pm$ 1,65 | 19,78 $\pm$ 2,20 | 27,35 $\pm$ 3,96 |
| **F2** | 19,66 $\pm$ 2,54 | 14,63 $\pm$ 0,86 | 8,37 $\pm$ 0,74 |
| **F3** | 32,27 $\pm$ 1,37 | 19,60 $\pm$ 3,00 | 12,36 $\pm$ 0,94 |
| **F4** | 69,41 $\pm$ 0,20 | 50,51 $\pm$ 1,83 | 75,60 $\pm$ 1,19 |
| **F5** | 53,9 $\pm$ 0,68 | 27,76 $\pm$ 3,34 | 49,32 $\pm$ 1,58 |
| **Amphotericin B** | 40,06 $\pm$ 1,17 | 42,51 $\pm$ 2,00 | 35,12 $\pm$ 2,61 |

**Biological tests with fractions F4 and F5**

Since it was noted that fractions F4 and F5 showed the best death results on the different *Leishmania species,* they were selected to continue the experiments. Their effective concentrations to disable 50% of the parasites were calculated and shown in Table 8.

**Table 8:** Effective concentration ($EC_{50}$) of F4 and F5 fractions of the aqueous extract of fungus *A. blazei* on promastigote forms of *Leishmania* species

| Promastigotes | $EC_{50}$ F4 (μg/mL) | $EC_{50}$ F5 (μg/mL) |
|---|---|---|
| *L. amazonensis* | 23,5 ($\pm$ 1,5) | 36,5 ($\pm$ 3,3) |
| *L. chagasi* | 25,1 ($\pm$ 2,0) | 38,7 ($\pm$ 2,8) |
| *L. major* | 35,8 ($\pm$ 1,3) | 64,7 ($\pm$ 2,7) |

Peritoneal macrophages were infected with parasites in the proportion of 10 parasites for each cell and, then, treated with the fractions F4 ad F5. The results are shown in figure 8. In order to investigate if the mechanism of leishmanicidal activity of the macrophages after stimulation with the purified fractions occurred by production of nitric oxide (NO), infected or non-infected macrophages were stimulated with fractions F3, F4 and F5 and the production of NO was determined by the Griess reaction. The results are shown in figure 9.

Also, the cytotoxicity of fractions F4 and F5 in mammalian cells was analyzed and the results are shown in Figure 10. To this end, macrophages were obtained from BALB /c and treated for 24 h at 25°C with fractions F4 and F5. Cell viability was assessed by MTT assay.

**Example 3 - Purification of fraction F5 obtained from the aqueous extract of fungus *Agaricus blazei***

After defining the use of fractions F4 and F5 for the experiments cited above, since they had presented the best indicators of leishmanicidal activity and did not show any significant cytotoxicity to mammalian macrophages, we decided to elect fraction 25 for continuing the experiments by the fact that it has a higher final yield after purification.

Thus, the purification in FPLC system of fraction F5 was performed and new, purest fractions were obtained. For this purpose, a pool of fraction F5 was eluted using a ion exchange MonoQ HR 5/5 column as fixed phase and NaCl solution as mobile phase. The samples were subjected to treatment against a NaCl concentration gradient (0 to 1M), collected, dialyzed and lyophilized.

They were also quantified and subjected to a protein electrophoresis in SDS-PAGE systems at 10% (Figure 11).

The new fractions obtained from F5 were tested as to their leishmanicidal activity on stationary phase promastigote forms of L. amazonensis and the results are shown in Figure 12. The drug amphotericin B at a concentration of 50 μg/mL was used as control. The experiment conducted by the cell viability protocol using the MTT reagent, and it was used the concentration of 20 μg/mL of the fractions.

It can be noted a high leishmanicidal activity in some of the new fractions purified by ion exchange, such as fractions F2, F3, F18, F19 and F27. Said fractions, even in a lower concentration than the previous fraction F5, have shown a greater activity than fraction F5. The fractions purified by ion exchange were also tested for toxicity (Figure 13) and presented no significant cytotoxicity.

**Example 4 - *In vivo* treatment of BALB/c mice infected with *Leishmania* amazonensis and treated with aqueous extract and fraction F5 of *Agaricus blazei.***

Treatment of mice infected with *L. amazonensis* was performed using said aqueous extract of *A. blazei* and the fraction called F5.

There were also tested 2 types of treatments: A prophylactic treatment (called chemoprophylaxis) and conventional treatment.

Since *in vitro* assays have demonstrated that the aqueous extract was able to decrease the penetration of parasites in macrophages, an experimental chemoprophylaxis model was elaborated to assess whether it would be possible, with a pre-treatment with aqueous extract of *A. blazei* or the fraction F5, to reduce infection and prevent or inhibit disease development in BALB/c mice.

For the chemoprophylaxis model, animals were previously treated for 5 days with the aqueous extract or fraction F5 and infected with $5x10^5$ stationary phase promastigote forms of *L. amazonensis,* in the right footpad. After infection, the animals were treated for further 20 days with a single dose of 2 mg per day of the aqueous extract or fraction F5, orally by gavage.

For the conventional treatment models, animals were infected with $5x10^5$ stationary phase promastigote forms of *L. amazonensis* in the right footpad and, then, treated for 20 days with a single dose of 2 mg per day of the aqueous extract or fraction F5, orally by gavage.

As control, the treatment for the same period (20 days) with 1 mg/Kg/day of the drug amphotericin B (deoxycholate) was used and, as infection control, some animals were infected and received saline for 20 days.

After infection, weekly measurements of the paws of infected animals were performed in order to monitor the progression of disease (Figure 13). The sacrifice of animals was performed at 10 weeks after infection, and some organs such as spleen, lesion and popliteal lymph node were collected and processed for parasitological and immunological analyzes.

In the analysis of Figure 14, it can be observed a significant reduction in swelling of the paws of animals from both proposed treatments (chemoprophylaxis and conventional treatment) with the aqueous extract of the fungus when compared to the swelling of the paws of infected animals who received saline. It is also observed that the reduction of the swelling in the paws of infected animals was even, in some measurements, less than that of the animals treated with amphotericin B.

In the analysis of Figure 15 it can be observed a significant reduction in swelling of the paws of animals subjected to treatment with fraction F5 when compared with the swelling of the paws of animals who received saline (infection control). Said reduction has even presented similar values to that observed in the infected paws of the animals treated with amphotericin B. The chemoprophylaxis showed no visible reduction of the swelling in the paws of infected animals when compared to infection control.

However, it should be noted that the animals that were undergoing chemoprophylaxis showed no organ toxicity, a fact that was observed in infected animals and those treated with amphotericin B, especially, significant increase of liver inflammation enzymes in these animals.

In the 10th week of infection, the animals were sacrificed for analysis of quantification of parasites, as shown in figures 16 and 17.

As observed in Figure 15, the conventional treatment and chemoprophylaxis with aqueous extract were able to significantly reduce the number of parasites in the evaluated organs (spleen, popliteal lymph node and infected paw) of infected animals, said reductions being greater when compared to the values found in the animals treated with amphotericin B.

Although the chemoprophylaxis model have shown good results when compared with the infection control (animals treated with saline), the best results were obtained with the conventional treatment, so that in this case there were found no parasites in the spleen of these animals and just a small number of parasites in the draining lymph nodes and infected paws, demonstrating the significant protection offered by the treatment with aqueous extract of *A. blazei.*

Despite the swelling of the paws of animals infected and that underwent chemoprophylaxis with fraction F5 did not provide significantly different values when compared to the control group, it was observed a significant reduction in the number of parasites in the animals that underwent chemoprophylaxis when compared to the infection control (Figure 16). It was also noted a significant reduction in the number of parasites in animals infected and treated with fraction F5, so that it was also not possible to detect parasites in the spleen of said animals. Thus, both chemoprophylaxis and conventional treatment with fraction F5 have shown significant reduction in the number of parasites in the different evaluated organs, said reductions being greater even related to the values observed in the animals treated with amphotericin B.

Thus, and conclusively, we have demonstrated that the aqueous extract of the fungus *A. blazei* and the fraction called F5 have an important leishmanicidal activity *in vivo* in BALB/c mice infected with *L. amazonensis.*

It should be noted that said studies were conducted twice and the results were similar. Presently, experiments in BALB/c mice infected with *L. chagasi* are in progress using the aqueous extract and fraction F5 and the results are promising both in chemoprophylaxis and in the conventional treatment of infected animals.

**Claims**

1. **LEISHMANICIDAL FORMULATION, characterized in that** it comprises the aqueous extract of mushroom *Agaricus blazei* and/or its purified fractions denominated F4 and F5, with corresponding proteins of 50 to 80 kDa, as well as non-protein components present in said products, such as tannins, saponins and polysaccharides, and at least one pharmaceutically acceptable excipient in liquid, semi-liquid or solid pharmaceutical forms.

2. **LEISHMANICIDAL FORMULATION** according to claim 1, **characterized in that** it is preferably used in pharmaceutical forms selected from the group consisting of gel, cream, ointment, pastes, emulsions in general, solutions, tablets and capsules.

3. **LEISHMANICIDAL FORMULATION** according to claims 1 and 2, **characterized in that** it is administered by oral and/or topical route.

4. **USE OF THE LEISHMANICIDAL FORMULATION,** according to claims 1 to 3, **characterized in that** it is used for the preparation of medicaments to prevent infection or treat mammalian infected with *Leishmania.*

5. **USE OF THE LEISHMANICIDAL FORMULATION,** according to claims 1 to 4, **characterized in that** it is applied in the prevention or in the clinical treatment of dogs and humans against Leishmaniasis.

Figure 1

Figure 2

## Figure 3

*L. amazonensis* 0 ug de Ab

*L. chagasi* 0 ug de Ab

*L. major* 0 ug de Ab

*L. amazonensis* 200 ug de Ab

*L. chagasi* 200 ug de Ab

*L. major* 200 ug de Ab

## Figure 4

Figure 5

L. amazonensis    L. chagasi    L. major

Figure 6

PM   F1   F2   F3   F4   F5   PM   F1   F2   F3

Figure 7

L. amazonensis promastigote
L.major promastigote
L.chagasi promastigote

concentration (50 µg/mL)

Figure 8

Amastigote intramacrophage
L. amazonensis

concentration (50 µg/mL)

Figure 9

Fractions in concentration of 50µg/mL

Figure 10

## Figure 11

## Figure 12

Figure 13

Figure 14

Figure 15

Figure 16

Figure 17

## INTERNATIONAL SEARCH REPORT

| International application No. |
|---|
| PCT/BR2011/000147 |

### A. CLASSIFICATION OF SUBJECT MATTER

**A61K36/07 (2006.01), A61P33/02 (2006.01)**

According to International Patent Classification (IPC) or to both national classification and IPC

### B. FIELDS SEARCHED

Minimum documentation searched (classification system followed by classification symbols)

**A61K, A61P**

Documentation searched other than minimum documentation to the extent that such documents are included in the fields searched

Electronic data base consulted during the international search (name of data base and, where practicable, search terms used)

**EPODOC, MEDLINE, WPI DATA, BIOSIS Previews, Chemical Abstracts, PASCAL Database**

### C. DOCUMENTS CONSIDERED TO BE RELEVANT

| Category* | Citation of document, with indication, where appropriate, of the relevant passages | Relevant to claim No. |
|---|---|---|
| Y | WO 9920258 A1 ( CRAVER WILLIAM E III [US]) 29 April 1999 (1999-04-29) See the whole document | 1 a 4 |
| Y | WO 2005065063 A2 (HETLAND GEIR [NO]) 21 July 2005 (2005-07-21) See the whole document | 1 a 4 |
| Y | WO 2008039169 A2 ( HIROMOTO BRYAN [US]) 03 April 2008 (2008-04-03) See the whole document | 1 a 4 |
| A | JP 1228480 A (NIPPON HYPOX LAB INC) 12 September 1989 (1989-09-12) | |

| [X] Further documents are listed in the continuation of Box C. | [X] See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 July 2011** | **26 September 2011** |

| Name and mailing address of the ISA/ | Authorized officer |
|---|---|
| INSTITUTO NACIONAL DA PROPRIEDADE INDUSTRIAL Rua Mayrink Veiga n° 9, 18° andar cep: 20090-050, Centro - Rio de Janeiro/RJ | **Welington Inácio de Almeida** |
| Facsimile No. | Telephone No. +55 21 3039-3493/3742 |

Form PCT/ISA/210 (second sheet) (July 2009)

# EP 2 570 131 A1

## INTERNATIONAL SEARCH REPORT

| | International application No. |
|---|---|
| | PCT/BR2011/000147 |

C (Continuation). DOCUMENTS CONSIDERED TO BE RELEVANT

| Category* | Citation of document, with indication, where appropriate of the relevant passages | Relevant to claim No. |
|---|---|---|
| A | JP 11263732 A (ICHIMARU PHARCOS INC) 28 **September** 1999 (1999-09-28) | |
| A | JP 11279204 A (SUMITOMO FORESTRY) 12 **october** 1999 (1999-10-12) | |
| A | JP 2000032946 A (PAWAFURU KENKO SHOKUHIN KK) 02 **February** 2000 (2000-02-02) | |
| Y | ROSA, L.H. et al. 'Cytotoxic, immunosuppresive, trypanocidal and leishmanial activities of Basidiomycota fungi present in Atlantic Rainforest in Brazil'. *Antonie van Leeuwenhoek*, 2009, vol. 95, página 227-237. See the whole document | 1 a 4 |
| A | ROSA, L.H. & CAPELARI, M. 'Agaricales fungi from Atlantic rain forest fragments in Minas Gerais, Brazil' *Brazilian J. Microbiol.* 2009, vol. 40, **pages** 846-851. | |
| A | SILVA, F.S. et al. 'In vitro pharmacological screening of macrofungi extracts from the Brazilian northeastern region' *Pharmaceutical Biology* 2009, vol. 47, 5, **pages** 384-389 | |
| A | BADALYAN, S.M. 'Antiprotozoal activity and mitogenic effect of mycelium of culinary-medicinal shiitake mushroom *Lentinus edodes* (Berk.) Singer (Agaricomycetideae)'. *International Journal of Medicinal Mushroom* 2004, vol. 6, **pages** 131-138. | |
| A | INCHAUSTI, A. et al. 'Leishmanicidal and trypanocidal activity of extracts and secondary metabolites from Basidiomycetes'. *Phytotherapy Research*, 1997, vol. 11, **pages** 193-197 | |
| A | YUEXIN, L. et al. 'Fractionation and characterization of water-soluble polysaccharides from culinary-medicinal mushroom, *Agaricus blazei* Murril (Agaricomycetideae)'. *International Journal of Medicinal Mushrooms*, 2002, vol. 4, **pages** 313-319. | |
| P, X | VALADARES, D.G. et al. 'Leishmanicidal activity of the Agaricus blazei Murill in different Leishmania species' *Parasitology International* (in press). Available online 24 June 2011 doi:10.1016/j.parint.2011.06.001 See the whole document | 1 a 4 |
| P, X | VALADARES, DG et al.'Leishmanicidal activity of *Agaricus blazei* fungus against different leishmania species'. Resumo PI026. Imuno 2010 - XXXV Congress of the Brazilian Society for Immunology. Porto Alegre, Rio Grande do Sul – Brazil. 03 a 06 de novembro de 2010 See the whole document | 1 a 4 |

Form PCT/ISA/210 (continuation of second sheet) (July 2009)

23

# EP 2 570 131 A1

<table>
<tr><td>INTERNATIONAL SEARCH REPORT</td><td>International application No.<br>PCT/BR2011/000147</td></tr>
</table>

**Box No. II    Observations where certain claims were found unsearchable (Continuation of item 2 of first sheet)**

This international search report has not been established in respect of certain claims under Article 17(2)(a) for the following reasons:

1. **[X]** Claims Nos.:    **5**
   because they relate to subject matter not required to be searched by this Authority, namely:
   **Methods for treatment of the human or animal body by surgery or therapy: PCT Article 17(2)(a)(i), PCT Rules 39.1(iv), 67.1(iv). However, since said method involves the administration to a human or animal patient, of a formulation obtained from an extract, the latter was used as the basis for the search of prior art documents.**

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**    [ ] The additional search fees were accompanied by the applicant's protest and, where applicable, the payment of a protest fee.
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (July 2009)

24

INTERNATIONAL SEARCH REPORT
Information on patent family members

International application No.

PCT/BR2011/000147

| | | | |
|---|---|---|---|
| WO 9920258 A1 | 1999-04-29 | AU 1105199 A | 1999-05-10 |
| | | CA 2308588 A1 | 1999-04-29 |
| | | EP 1024796 A1 | 2000-08-09 |
| | | US 5906825 A | 1999-05-25 |
| WO 2005065063 A2 | 2005-07-21 | CN 1953759 A | 2007-04-25 |
| | | EP 1708673 A2 | 2006-10-11 |
| | | JP 2007517867 A | 2007-07-05 |
| | | NO 20044581 D0 | 2004-10-25 |
| | | RU 2006129301 A | 2008-02-20 |
| | | US 2008233144 A1 | 2008-09-25 |
| | | WO 2005065063 A3 | 2005-11-17 |
| WO 2008039169 A2 | 2008-04-03 | CA 2618096 A1 | 2007-02-01 |
| | | CN 101389764 A | 2009-03-18 |
| | | EP 1931360 A2 | 2008-06-18 |
| | | JP 2009513579 A | 2009-04-02 |
| | | RU 2008108023 A | 2009-09-10 |
| | | US 2011136758 A1 | 2011-06-09 |
| | | WO 2008039169 A3 | 2008-11-13 |
| | | ZA 200801558 A | 2009-10-28 |
| JP 1228480 A | 1989-09-12 | EP 0413053 A1 | 1991-02-20 |
| JP 11263732 A | 1999-09-28 | NONE | |
| JP 11279204 A | 1999-10-12 | NONE | |
| JP 2000032946 A | 2000-02-02 | NONE | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **WASSER, S.P.** Medicinal mushrooms as a source of antitumor and immunomodulating polysaccharides. *Appl. Microbiol. Biotechnol.,* 2002, vol. 60, 258-74
- **BRASILIA, DF.** *International Symposium on mushrooms in Brazil,* 2004, 78-80
- **STIJVE, T. ; AMAZONAS, M.A.L. ; GILLER, V.** Flavor and taste components of Agaricus blazei ss. Heinem: a new gourmet and medicinal mushroom. *Deutsche Lebensmittel-Rundschau, Stuttgart,* 2002, vol. 98, 448-453
- **MIZUNO, M. ; MORIMOTO, M. ; MINATO, K. ; TSUCHIDA, H.** Polysaccharides from Agaricus blazei stimulate lymphocyte T-cell subsets in mice. *Biosci. Biotechnol. Biochem.,* 1998, vol. 62, 434-437
- **URBEN, A.F.** Morphological and physiological characterization in accesses of Agaricus blazei and A. sylvaticus. *V Latin American Mycology Congress, Brasilia,* 2005, 203-205
- **WASSER, S.P. ; WEIS, A.L.** Medicinal properties of substances occurring in higher basidiomycetes mushrooms: Current perspectives (review. *Int. J. Med.,* 1999, vol. 1, 31-62
- **MIZUNO, T. ; HAGIWARA, T.** Antitumor activity and some properties of water-soluble polysaccharides from "Himematsutake", the fruiting body of Agaricus blazei Murrill. Agricult. *Biologic. Chemist., Tokyo,* 1990, vol. 54, 2889-2896
- **MIZUNO, T. ; INAGAKI, R. ; KANAO, R.** Antitumor activity and some properties of water-insoluble hetero-glycans from "Himematsutake", the fruiting body of Agaricus blazei Murill. *Agric. Biol. Chem.,* 1990, vol. 54, 2897-2905
- **EBINA, T. ; FUJIMIYA, Y.** Antitumor effect of a peptide-glucan preparation extracted from Agaricus blazei in a double-grafted tumor system in mice. *Biotherapy,* 1998, vol. 11, 259-65
- **KUO, Y.C. ; HUANG, Y.L. ; CHEN, C.C.** Cell cycle progression and cytokine gene expression of human peripheral blood mononuclear cells modulated by Agaricus blazei. *J. Lab. Clin. Med.,* 2002, vol. 140, 176-87
- **DONG Q ; YAO J ; YANG XT ; FANG JN.** Structural characterization of a water-soluble beta-D-glucan from fruiting bodies of Agaricus blazei Murr. *Carbohydr Res.,* 2002, vol. 3, 1417-21
- **FUJIMIYA Y ; SUZUKI Y ; OSHIMAN K.** Selective tumoricidal effect of soluble proteoglucan extracted from the basidiomycete, Agaricus blazei Murill, mediated via natural killer cell activation and apoptosis. *Cancer Immunol Immunother.,* 1998, vol. 46, 147-59
- **NAKAJIMA, A. ; ISHIDA, T. ; KOGA M.** Effect of hot water extract from Agaricus blazei Murill on antibody-producing cells in mice. *Int. Immunopharmacol.,* 2002, vol. 2, 1205-11
- **SHIMIZU, S. ; KITADA, H. ; YOKOTA, H.** Activation of the alternative complement pathway by Agaricus blazei murill. *Phytomedic.,* 2002, vol. 9, 536-45
- **SORIMACHI, K. ; AKIMOTO, K. ; IKEHARA, Y.** Secretion of TNF-$\alpha$, Il-8 and nitric oxide by macrophages activated with Agaricus blazei murrill fractions in vitro. *Cell Struct. Func.,* 2001, vol. 26, 103-108
- **NAKAJIMA, A. ; ISHIDA, T. ; KOGA M.** Effect of hot water extract from Agaricus blazei Murill on antibody-producing cells in mice. *Int Immunopharmacol.,* 2002, vol. 2, 1205-11
- **SAKAGAMI, H. ; AOKI, T. ; SIMPSON, A.** Induction of immunopotentiation activity by a protein-bound polysaccharide, PSK (Review. *Anticancer Res.,* 1991, vol. 11, 993-1000
- **SORIMACHI, K. ; NIWA, A. ; YAMAZAKI, S.** Antiviral activity of water-solubilized lignin derivatives in vitro. *Agric. Biol. Chem.,* 1990, vol. 54, 1337-1339
- **OOI, V.E.C.** Hepatoprotective effect of some mushrooms. *Phytother. Res. West Sussex,* 1996, vol. 10, 536-538
- **PARK, E.J. ; KO, G. ; KIM, J.** Antifibrotic effects of a polysaccharide extracted from Ganoderma lucidum, glyclyrrhizin, and pentoxifyline in rats with cirrhosis induced in biliary obstruction. *Biol. Pharm. Bull.,* 1997, vol. 20, 417-420
- **HIKINO, H. ; MIZUNO, T.** Hypoglycemic actions of some heteroglycans of Ganoderma lucidum fruit bodies. *plant Med.,* 1989, vol. 55, 385
- **CHEUNG, P.C.K.** The hypocholesterolemic effect of extracellular polysaccharide from the submerged fermentation of mushroom. *Nutr. Res.,* 1996, vol. 16, 1953-1957
- **DESJEUX, P.** Leishmaniasis: current situation and new perspectives. *Comp. Immunol. Microbiol. Infect. Dis.,* 2004, vol. 27, 305-318

- **GRIMALDI, G.JR. ; TESH, R.B.** Leishmaniasis of the New World: current concepts and implications for future research. *Clin. Microbiol. Res.,* 1993, vol. 6, 230-250
- **SACKS, D. ; KAMHAWI, S.** Molecular aspects of parasite-vector and vector-host interactions in leishmaniasis. *Annu. Rev. Microbiol.,* 2001, vol. 55, 453-483
- **GRIMALDI, G.JR. ; TESH, R.B.** Leishmaniasis of the New World: current concepts and implications for future research. *Clin. Microbiol. Rev.,* 1993, vol. 6, 230-250
- **SACKS, D. ; SHER, A.** Evasion of innate immunity by parasitic protozoa. *Nat. Immunol.,* 2002, vol. 3, 1041-1047
- **MARZOCHI, M.C. ; MARZOCHI, K.B. ; CARVALHO, R.W.** Visceral leishmaniasis in Rio de Janeiro. *Parasitol. Today,* 1994, vol. 10, 34-37
- **DESJEUX, P.** Leishmaniasis: current situation and new perspectives. Comp. *Immunol. Microbiol. Infect. Dis.,* 2004, vol. 27, 305-318
- **SILVEIRA FT ; LAINSON R ; CORBETT CE.** Clinical and immunopathological spectrum of American cutaneous leishmaniasis with special reference to the disease in Amazonian Brazil: a review. *Mem. Inst. Oswaldo Cruz. V.,* 2004, vol. 99, 239-251
- **WEIGLE, K. ; SARAVIA, N.G.** Natural history, clinical evolution, and the host-parasite interaction in New World cutaneous leishmaniasis. *Clin. Dermatol.,* 1996, vol. 14, 433-450
- **SHAW, J.** The leishmaniasis-survival and expansion in a changing world. *A mini-review. Mem. Inst. Oswaldo Cruz.,* 2007, 541-7
- **CARVALHO, P.B. ; ARRIBAS, M.A.G. ; FERREIRA, E.I.** Leishmaniasis. What do we know about its chemotherapy?. *Braz. J. Pharmac. Sci.,* 2000, vol. 36, 69-96
- **FRANKE, E.D. ; WIGNALL, F.S. ; CRUZ, M.E. ; ROSALEZ, E. ; TOVAR, A.A. ; LUCAS, C.M. ; LIANOS-CUENTAS, A. ; BERMAN, J.D.** Efficacy and toxicity of sodium stibogluconate for mucosal leishmaniasis. *Ann. Intern. Med.,* 1990, vol. 113, 934-940
- **HERWALDT, B.L.** Leishmaniasis. *Lancet,* 1999, vol. 354, 1191-1199
- **SUNDAR, S. ; SINGH, A. ; AGARWAL, D. ; RAI, M. ; AGARWAL, N. ; CHAKRAVARTY, J.** Safety and efficacy of high-dose infusions of a preformed amphotericin B fat emulsion for treatment of Indian visceral leishmaniasis. *Am. J. Trop. Med. Hyg.,* 2009, vol. 80, 700-3
- **GROGL, M. ; MARTIN, R.K. ; ODUOLA, A.M.J. ; MILHOUS, W.K. ; KYLE, D.E.** Characteristics of multidrug resistance in Plasmodium and Leishmania: detection of P-glycoprotein-like components. *Am. J. Trop. Med. Hyg.,* 1991, vol. 45, 98-111
- **TAVARES CA ; FERNANDES AP ; MELO MN.** Molecular diagnosis of leishmaniasis. *Expert Rev Mol Diagn.,* 2003, vol. 3, 657-667
- **CUNNINGHAM, A.C.** Parasitic adaptive mechanisms in infection by Leishmania. *Exp. Mol. Pathol.,* 2002, vol. 72, 131-141